# EUROPEAN PATENT SPECIFICATION

(11) **EP 1 238 714 B1**
(45) Date of publication and mention of the grant of the patent: **02.03.2005**
(21) Application number: 02076507.9
(22) Date of filing: 12.12.1997
(51) Int. Cl.: B05D 7/00, A01C 1/06, A61K 7/06, D06M 15/263, D06M 23/00, C08F 2/18

(54) **Aqueous dispersions of crystalline polymers and their use**
Wässrige Emulsionen von kristallinen Polymeren und ihre Verwendung
Dispersions aqueuses de polymères cristallins et leur utilisation

(30) Priority: 12.12.1996 US 766865; 12.12.1996 US 769639; 15.09.1997 US 929750
(43) Date of publication of application: 11.09.2002
(62) Divisional of application: 97953166.2
(73) Proprietor: LANDEC CORPORATION, Menlo Park California 94025 (US)
(72) Inventor: Stewart, Ray F., Redwood City, California 94062 (US); Balachander, Natarajan, Sunnyvale, California 94085 (US); Yoon, Valentine Y., Santa Barbara, California 93111 (US); Bitler, Steven P., Menlo Park, California 94025 (US); Phan, Loc, San Jose, California 95131 (US)
(74) Representative: Hall, Robert Leonard

(56) References cited:
- WO-A-92/10081
- WO-A-93/21967
- WO-A-96/27641
- US-A- 4 104 824
- US-A- 4 184 020
- CHEMICAL ABSTRACTS, vol. 103, no. 24, 16 December 1985 (1985-12-16) Columbus, Ohio, US; abstract no. 196567c, HITACHI CHEMICAL CO LTD.: "Granular polymers" page 11; XP002063723 & PATENT ABSTRACTS OF JAPAN vol. 9, no. 271 (C-311), 29 October 1985 (1985-10-29) & JP 60 120702 A (HITACHI KASEI KOGYO KK), 28 June 1985 (1985-06-28)
- CHEMICAL ABSTRACTS, vol. 112, no. 20, 14 May 1990 (1990-05-14) Columbus, Ohio, US; abstract no. 180099b, TAYAMA S. ET AL: "Method for suspension polymerization" XP000063301 & PATENT ABSTRACTS OF JAPAN vol. 14, no. 24 (C-677), 18 January 1990 (1990-01-18) & JP 01 266104 A (MITSUBISHI RAYON CO. LTD.), 24 October 1989 (1989-10-24)
- CHEMICAL ABSTRACTS, vol. 119, no. 24, 13 December 1993 (1993-12-13) Columbus, Ohio, US; abstract no. 250751d, IMAMURA TAKESHI ET AL.: "Preparation of polymer particles with narrow particle size by suspension polymerization" page 19; XP000407439 & PATENT ABSTRACTS OF JAPAN vol. 17, no. 499 (C-1109), 9 September 1993 (1993-09-09) & JP 05 132504 A (NIPPON PAINT CO LTD.), 28 May 1993 (1993-05-28)

## Description

This invention relates to aqueous dispersions of crystalline polymers, methods for making such dispersions and the use of such dispersions for coating human hair.

It is known to use certain organic polymers to provide coatings on a wide variety of substrates, including hair (see U.S. Pat. Nos. 4,196,190; 4,452,216; 4,834,968 and 4,983,383); fabrics (see U.S. Patent Nos. 3,644,241; 3,833,393 and 4,780,499); and seeds and other organisms (see U.S. Patent No. 5,129,180). It is also known to set a fabric by impregnating the fabric with a monomer component, and polymerizing the monomer while maintaining the fabric in a desired configuration (see U.S. Patent No. 4,401,688).

One class of polymers which has been used for coating substrates comprises side-chain crystalline ("SCC") polymers. However, the known coating compositions containing SCC polymers have the disadvantage that the polymer is dissolved in an organic solvent. Organic solvents are expensive, require environmental controls, and are particularly undesirable for application to human hair or to an organism.

Attempts have been made to prepare aqueous dispersions of SCC polymers (see *British Polymer Journal* 20, 521-524 (1988), U.S. Patent Nos. 5,506,307 and 5,516,865, and Japanese Patent Document No. 6-192341), but the results have been poor and/or the techniques expensive.

We have discovered, in accordance with the present invention, that very valuable results can be achieved by coating human hair with aqueous dispersions of polymeric particles in which the polymer is a crystalline polymer. The matter for which protection is sought is defined in the appended claims.

In describing the invention below, unless otherwise noted, parts, percentages and ratios are by weight. The monomers from which the crystalline polymer is derived are important. The term hydrophobic monomer is used herein to denote a monomer whose solubility in water at 25°C is less than 2%. The term hydrophilic monomer is used herein to denote a monomer whose solubility in water at 25° is 2% or more. More than one hydrophobic monomer can be used, and the term hydrophobic monomer component is used herein to denote all the hydrophobic monomers collectively, and is often abbreviated herein to "BMC." More than one hydrophilic monomer component can be used, and the term hydrophilic monomer component is used herein to denote all the hydrophilic monomers collectively, and is often abbreviated herein to "PMC." The monomers from which the crystalline polymer is derived can include one or more monomers which contain two or more polymerizable groups, so that the polymer is crosslinked. Such monomers (which may be hydrophobic or hydrophilic) are referred to herein as crosslinking monomers; and the term crosslinking monomer component is used herein to denote all the crosslinking monomers collectively, and is often abbreviated herein to "XLC." Except where otherwise noted, percentages of monomers given herein (both as starting materials, and as residual monomers) are based on the total weight of the monomer starting material. As further discussed below, the monomers used in preparing the crystalline polymers are preferably n-alkyl acrylates or methacrylates. The abbreviation CnA and 'CnMA are used to denote n-alkyl acrylates and n-alkyl methacrylates, respectively, in which the n-alkyl group contains x carbon atoms. For example C4A denotes butyl acrylate, and C12MA denotes lauryl methacrylate.

Crystalline melting points given herein (Tₘ and T_{c}) are the peak of a curve produced using a differential scanning calorimeter (DSC) at a heating rate of 10°C per minute, and using the second heat cycle. The onset of melting (Tₒ) is taken from the same curve. Tack values given herein are measured as follows. The latex is applied to a polymeric film (a polyester film available from DuPont under the trade name "Mylar," or a filled polyolefin film available from PPG under the trade name "Teslin"), at a rate such that after the coating has been dried at 65°C for 2 hours, it has a thickness of about 30 microns. Two such films are laminated together at 35°C and the tack is measured with a vslip/peel tester at 230 cm/min

### A. Materials for Use In The Preparation of the Polymeric Dispersions

In order to prepare the aqueous polymeric dispersions which form part of the present invention, and/or which can be used to coat substrates in accordance with the invention, a monomer component is dispersed in water with the aid of a surfactant and preferably a cosolvent; optionally other additives may also be present. The reaction mixture is then subjected to conditions which cause the monomer(s) to react to form a polymer which remains in suspension in the reaction medium. The monomer component comprises a hydrophobic monomer component (BMC) and a hydrophilic monomer component (LMC). Either the BMC or the LMC, or both, can include a crosslinking monomer. The reaction mixture can also contain a crosslinking agent, which can be (a) a crosslinking monomer component (XLC) which comprises at least one monomer containing at least two polymerizable groups, or (b) an "external crosslinker." An external crosslinker is a compound which increases the apparent molecular weight of the polymer without becoming a covalently bound part of the polymer, and which increases the mechanical strength of coatings formed from the aqueous dispersion of the polymer.

### 1. Monomer Components

### (a) Hydrophobic Monomer Component (BMC)

The BMC comprises at least one first hydrophobic monomer such that a homopolymer thereof is crystalline and has a melting point T_{c}. The BMC includes at least one second hydrophobic monomer such that a homopolymer thereof is amorphous and has a glass transition point T_{g} which is less than T_{c} preferably, the second hydrophobic monomer is present in amount such that the Tₘ of the polymer is (T_{c} -5)°C or less. The amount of the second hydrophobic monomer is generally less than 50%, preferably 5 to 40%, particularly 5 to 30%, based on the BMC.

Preferred hydrophobic monomers are α, β-ethylenically-unsaturated monomers, particularly n-alkyl acrylates and n-alkyl methacrylates in which the n-alkyl groups contains 4 to 24 carbons.

The first hydrophobic monomers are monomers whose homopolymers are side chain crystalline (SCC) polymers. SCC polymers are well known, and are described for example in the following publications, to which reference should be made for a more complete disclosure of first hydrophobic monomers suitable for use in this invention: J. Poly. Sci. 60, 19 (1962), J. Poly. Sci. (Polymer Chemistry) 7, 3053 (1969), 9, 1835, 3349, 3351, 3367, 10, 1657, 3347, 18, 2197, 19, 1871, J. Poly. Sci., Poly Physics Ed 18 2197 (1980), J. Poly. Sci. Macromol. Rev. 8, 117 (1974), Macromolecules 12, 94 (1979), 13, 12, 15, 18, 2141, 19, 611, JACS 75, 3326 (1953), 76, 6280, Polymer J 17, 991 (1985), Poly. Sci USSR 21, 241 (1979), US Patent Nos. 4,380,855, 5,120,349, 5,129,180, 5,156,411, 5,254,354, 5,387,450, 5,412,035, 5,469,869, and 5,665,822. Particularly preferred first hydrophobic monomers are those which result in SCC polymers in which the side chain comprises a linear polymethylene group containing 12 to 50, preferably 14 to 22, carbons, or a linear perfluorinated or substantially perfluorinated polymethylene group containing 6 to 50 carbons. Mixtures of such hydrophobic monomers can be used. Preferred monomers are those which are commercially available, in particular the n-alkyl acrylates. Preferred first hydrophobic monomers for use in the invention comprise at least one of C12MA, C12A, C14A, C14MA, C16A, C16M, C18A, mixtures of C18A, C20A and C22A, mixtures of C26A and C40A, fluorinated C8A, and mixtures of fluorinated C8A, C10A and C12A, and other monomers which produce substantially the same final polymers, e.g. acrylamides and methacrylamides.

The second hydrophobic monomers are monomers whose homopolymers have a T_{g} of -50 to 120°C, for example n-butyl, isobutyl, and tertiary butyl methacrylates, vinyl acetate, vinyl propionate, n-butyl and t-butyl acrylates, homopolymers of which have melting points of 32, 65, 107, 28, 10, 40, and 52°C, respectively. Examples of suitable second hydrophobic monomers are linear and branched chain alkyl, hydrohyalkyl and alkoxyalkyl acrylates, methacrylates, acrylamides, and methacrylamides, and vinyl esters, in particular C8A, C8MA, 2-ethylhexyl acrylate and methacrylate, C6A, C6MA, C4A, C4MA and mixtures thereof.

### (b) Hydrophilic Monomer Component (LMC)

The LMC comprises at least one hydrophilic monomer and is preferably present in an amount of 2 to 10%, particularly 2 to 5%. The hydrophilic monomer is preferably an αβ-ethylenically unsaturated monomer, for example a vinylic compound such as acrylic acid; methacrylic acid; an alkyl, hydroxyalkyl or alkoxyalkyl acrylate or methacrylate (e.g. hydroxy ethyl methacrylate); acrylamide and methacrylamide; acrylic or methacrylic acid; acrylamide; methacrylamide; maleic anhydride; itaconic acid; a comonomer containing an amine group; and 2-acrylamido-2-methylpropane sulfonic acid (AMPS) and combinations thereof. Preferably the hydrophilic monomer is selected from the group consisting of acrylic acid, methacrylic acid, itaconic acid, 2-acrylamido-2-methylpropane sulfonic acid, acrylamide, methacrylamide, or hydroxyethyl (meth)acrylate or a mixture thereof.

### (c) Crosslinking Monomer Component (XMC)

. The XMC, if present, comprises at least one crosslinking monomer which contains at least two polymerizable groups, e.g. vinylic groups. The XMC is preferably used in amount such that the polymer has a gel content of at least 50%, particularly at least 60%, up to substantially 100%. Preferred hydrophobic crosslinking monomers include 1,4-butanediol diacrylate, 1,6-hexanediol diacrylate, 1,14-tetradecyldiol diacrylate, divinyl benzene, vinyl crotonate, divinyl ether, and 1,3,5-triallyl triazine trione, and mixtures thereof. Preferred hydrophilic crosslinking monomers include ethylene glycol diacrylate, and ethylene glycol dimethacrylate, and mixtures thereof.

The amount of crosslinking monomer is preferably such that the polymer has a crosslink density (number of crosslinks per weight average molecular weight) of at least 0.01, preferably at least 0.05, particularly at least 0.1, so that the polymer resists flow above Tₘ. A high gel content (e.g. crosslinking beyond 1 to 10 mole percent) is not generally needed and can result in decreased crystallinity and poor performance. The crosslinked polymer preferably has a heat of fusion of at least 10, preferably at least 20, J/g.

The use of an XMC is important when it is desirable for the coating to be easily removed, because the resulting crosslinked polymer particles are harder; and when water is evaporated from dispersion, the particles do not easily diffuse each other. The resulting coatings have a particulate character and as a result are more easily removable and frangible. This is particularly important for hair-setting compositions, since it is desirable that the film-forming material be removable by washing hair with water. It is also particularly important for seed coatings; prior art non-particulate coatings can remain on the seed and inhibit seed germination. In the absence of crosslinking, when the water is evaporated, the latex particles are brought together by capillary action, and the water is squeezed out from the interstitial spaces. The particles coalesce and lose their identity, and a substantially uniform film results.

### (d) Carboxyl-Containing Monomer Component

The monomer component can contain one or more monomers containing carboxyl groups, the carboxyl groups being partially, preferably at least 75%, neutralized (e.g. with NaHCO₃) after the polymerization is complete.

### (e) Preferred Monomer Components

Preferred monomer components result in a crystalline polymer having a sharp melting point, preferably such that (Tₘ-Tₒ) is less than 20°C, preferably less than 15°C, particularly less than 10°C, where Tₒ is the onset of melting on the DSC curve.
Particularly preferred monomer components comprise:
(i) 50 to 100%, preferably 65 to 95%, of at least one first hydrophobic monomer which is an alkyl acrylate, alkyl methacrylate, alkyl acrylamide, alkyl methacrylamide, alkyl vinyl ether or alkyl vinyl ester in which the alkyl group is an n-alkyl group containing 12 to 50 carbon atoms;
(ii) 0 to 35% of at least one second hydrophobic monomer which is an alkyl acrylate, alkyl methacrylate, N-alkyl acrylamide, alkyl vinyl ether, or alkyl vinyl ester in which the alkyl group is an n-alkyl group containing 4 to 12 carbon atoms; and
(iii) at least 2%, and preferably no more than 10% of at least one hydrophilic monomer which is acrylic acid, methacrylic acid, 2-acrylamido-2-methylpropane sulfonic acid, hydroxyethyl acrylate or methacrylate itaconic acid, acrylamide, methacrylamide, acrylonitrile, methacrylonitrile, a tetraalkylammonium acrylate, vinyl acetate or N-vinyl pyrrolidone.

### 2. External Crosslinkers

External crosslinkers, when used, are generally compounds containing charged groups which interact with charged groups in the polymer. Preferred external crosslinkers contain metal (e.g. Mg, Mn, Zn, Ca, Ti or Zr) cations or bidentate or multidentate amine groups. Other external crosslinkers are non-metallic and organic compounds, e.g. triethylene tetramine, ethylene diamine, diethyl aminopropylamine and bis(quaternary ammonium) salts. The amount of external crosslinker is generally 0.1 to 10, preferably 0.5 to 3%, based on the monomer component. Some external crosslinkers do not have any effect until a coating is prepared from the dispersion. For example, the cation can be present as an ammonia complex and not become effective until the ammonia is removed during the evaporation step. Thus, an aqueous solution containing Zn(NH₃)⁺⁺ ions can be used in this way.

External crosslinkers can be used in combination with crosslinking monomers.

### 3. Surfactants

The reaction medium for the polymerization comprises at least one surfactant, preferably at least one ionic surfactant and at least one non-ionic surfactant, to suspend and stabilize the particles formed by polymerizing the monomer component in the aqueous reaction medium. The amount of surfactant can be 0.8 to 10%, preferably 1 to 5%, based on the reaction medium. However, the optimum amount of surfactant depends also on the amount of cosolvent present, as further described below. Preferably, sufficient surfactant is present to enable the monomer component to be present initially as droplets having a diameter of more than 10 microns.

Surfactants are characterized by a hydrophile-lipophile balance (HLB) number that describes the strength of the hydrophilicity to the lipophilicity of the surfactant. Anionic surfactants having a HLB number of 20 to 40 are preferably used, and function to form micelles at low concentrations and to act as nucleation sites for the latex particles. Once nucleation is complete, the surfactants can additionally provide charge stabilization to the growing latex particles. Preferably the anionic surfactants contain a sulfate or sulfonate group and a hydrophobic moiety and may be, for example, alkyl sulfates, alkyl ether sulfonates and sulfates, alkylaryl sulfonates, and sulfosuccinates. Preferred anionic surfactants are the amine salt of dodecyl benzene sulfonic acid, salts of dioctyl sulfosuccinate, salts of lauryl sulfate, and salts of the ethoxylated alcohol half ester of sulfosuccinic acid. Exemplary alkyl and alkene suifonates are described in U.S. Pat. Nos. 4,983,383 and 5,194,469.

Nonionic surfactants having HLB numbers of 5 to 35, e.g. 5 to 30, preferably 10 to 15, can also be employed. These surfactants lower the interfacial tension between the monomer oil droplets and the aqueous phase, and provide stabilization of the polymer dispersion by partitioning at the interface of the aqueous phase and the polymer particles. Thus they reduce the polymer dispersion's sensitivity to shear, temperature and the presence of electrolytes. The nonionic surfactants can be linear or branched ethoxylated alcohols, ethoxylated alkylphenols, or block copolymers of ethylene oxide and propylene oxide. Preferably they contain 3 to 40 ethylene oxide repeat units and a hydrophobic moiety which may be a linear or branched C₁₁-C₁₅ secondary alcohol, nonylphenol or octylphenol, or are block copolymers of ethylene oxide and propylene oxide.

The amount of surfactant present is preferably such that high energy homogenization of the monomer component is not needed to form the latex. When little or no cosolvent is used, preferably a relatively large amount of surfactant (e.g. at least 5% based on the monomer component) is used so that the monomer component is dispersed in the reaction mixture as droplets having a diameter greater than 10 microns.

The amount of surfactant and cosolvent in the reaction mixture is preferably such that the units derived from the second hydrophobic monomer (if present) and/or the hydrophilic monomer are substantially randomly dispersed in the polymer.

The surfactant can be a polymerizable component which is incorporated into the polymer.

The choice of the surfactant may be influenced by the end use of the dispersion. For example, particularly useful surfactants for compositions used for coating corn seeds are fluorine-containing surfactants.

### 4. Cosolvents

The reaction medium preferably contains at least one cosolvent (also referred to as a diffusion modifier) which increases the solubility of the hydrophobic monomer in the reaction medium. It is generally present in amount at least 10% but less than 30%, preferably 5 to 20%, based on the water. Because the cosolvent increases the water solubility of the hydrophobic monomer, it increases the transport rate of the hydrophobic monomer from the monomer micelles or oil droplets to polymer nucleation sites. The cosolvents are preferably linear or branched monoalkanols containing 1 to 4 carbons, alkyl esters or alkyl ethers containing 1 to 6 carbons. Preferred cosolvents have a solubility in water of more than 0.7% at 25° C and have boiling points of 60° to 130°C, preferably less than 95°C, so that they can be easily removed after the polymerization reaction. Preferred cosolvents include ethanol, isopropanol, ethyl acetate, n-propanol, sec-butanol, and t-butanol, and combinations thereof. Preferably the cosolvent has a water-octanol partition coefficient of at least 0.2 to enhance the solubility of the hydrophobic monomers in the water (see "Handbook of Property Estimation Methods," edited by W.J. Lyman, W.F. Reehl, D.H. Rosenblatt, American Chemical Society, Washington, D.C. 1990).

The amount of cosolvent preferably used depends upon the amount and type of surfactant used. For example, where there is about 1.6% of ionic and non-ionic surfactants, at least 5% of the cosolvent is generally required to obtain a single sharp melting point crystalline polymer, whereas when about 0.8% of ionic and non-ionic surfactants is present, at least about 10% of the cosolvent is generally required.

### 5. Initiators

A free radical initiator must generally be present to cause the polymerization to take place, generally in amount 0.1 to 0.4% based on the reaction mixture. Alternatively or additionally, ultraviolet radiation can be used to initiate the reaction.

Initiators useful in emulsion polymerization are well known [see, for example, U.S. Patent No. 5,516,865 and "Emulsion Polymerization," Blackley (1975), chapter 6, Applied Science Publishers, London]. Suitable hydrophobic initiators for this invention include benzoyl peroxide and di-t-butyl peroxide. Suitable hydrophilic initiators include hydrogen peroxide and potassium and ammonium persulfate. A redox initiator, e.g. t-butyl hydroperoxide/ascorbic acid, or hydrogen peroxide/ferrous compound, can also be used.

### 6. Other Additives

The reaction mixture can contain a variety of other additives, including pH adjusters (e.g. sodium hydroxide and ammonium hydroxide); pH buffers (e.g. sodium bicarbonate and sodium hydrogen phosphate); coalescing agents (e.g. N-methylpyrrolidone, poly(ethylene glycol) alkyl ethers and poly(ethylene glycol); colloidal stabilizers (e.g. hydroxyethyl cellulose, polyacrylates and polyvinyl alcohols); rheology modifiers (e.g. hydroxyethyl cellulose and the products available under the trade names Kelzan and VCAR from Kelco and Union Carbide, respectively); and defoamers (e.g. the products available under the trade names Surfynol and Anti-foam H-10 Emulsion from Air Products and Dow Corning, respectively. The addition of sodium bicarbonate tends to increase particle size.

### B. Polymerization Conditions

### 1. Single Stage Polymerization

The polymerization is generally carried out at 45 to 90°C, preferably 60 to 80°C. Lower temperatures decrease the amount of coagulum and increase particle size uniformity. Preferably the reaction is carried out under oxygen-free conditions, e.g. under nitrogen. Polymerization is preferably continued until at least 99% of the monomer component has been polymerized. Typically this takes 1 to 4 hours at about 70°C.

The polymerization is preferably carried out under conditions such that the polymer is obtained in the form of particles having an average size of less than 1 micron, preferably less than 0.5 micron, particularly 0.25 to 0.35 micron (measured by the well known light scattering technique). Small particle sizes are desirable because the dispersions are more stable, and do not clog spray nozzles. Preferably the amount of monomer is such that the reaction product has a solids content of at least 25%, particularly at least 30%, especially at least 35%. Higher solids content is desirable because less water needs to be evaporated to form a coating, and because the product is less expensive to package and transport. However, the solids content is generally no more than 45%, because of the tendency of very high solids content dispersions to coagulate spontaneously.

After the polymerization is complete, the cosolvent, if used, is generally removed. At this stage, further additives can be added to the latex, depending on the intended use of the product.

### 2. Two Stage Polymerization

In some applications it can be desirable in a first stage to polymerize the hydrophobic component thereby forming a crystalline copolymer or homopolymer, and then polymerizing the hydrophilic component in a second stage. This results in phase-separated particles having a core of predominantly (at least 50% by weight) crystalline polymer and a shell around the core, the shell being formed predominantly of non-crystalline polymer. Generally, the core is substantially all crystalline polymer as a first phase, and the shell is substantially all non-crystalline polymer as the shell. This is desirable when there is a need to change the properties of the crystalline polymer, such as tack, without changing the Tₘ. The monomers used to form the shell are not limited to hydrophilic materials, but can also be monomers such as styrene or butyl methacrylate.

For a core/shell product, Tₘ is substantially the same as the crystalline homopolymer formed from the first hydrophobic monomer.

As in the single stage polymerization, the final latex preferably has a solids content of at least 30% and a residual monomer content of less than 1 %; and the surfactant likewise preferably is present in amount such that the latex particles have an average particle size of less than 0.1 micron, particularly less than 0.5 micron.

### C. Treatment of human hair

Compositions for treating hair (particularly human hair, but also hair on a wig, or hair on a doll) preferably include an effective amount of at least one hair care additive, this term being used to denote any compound conventionally used in hair treating formulations. The amount of each such additive is preferably 0.01 to 10%, preferably 0.05 to 5%, based on the composition. Suitable additives include sequestering agents, hair-conditioning agents (e.g. cetyl trimethyl ammonium chloride, glyceryl monostearate, glycerin, propylene, glycol, sorbitol, urea, isopropyl myristate, balsam, lanolin, spermaceti, and isopropyl palmitate), anti-dandruff agents (e.g. sulfur, salicylic acid, resorcinol, bithionol, chloral hydrate, and zinc pyrithone), reducing agents (e.g. thioglycolates), bleaching agents (e.g. hydrogen peroxide, and perborate and persulfate salts), scents, perfumes, pearlescent aids (e.g. ethylene glycol distearate), preservatives (e.g. benzyl alcohol, methylparaben, propylparaben, imidazolidinyl urea, and esters of p-hydroxybenzoic acid), thickeners (also known as viscosity modifiers, e.g. methyl cellulose, starches and starch derivatives), coloring agents (e.g. any of the FD&C or D&C dyes), and pH adjusting agents (e.g. citric acid, sodium citrate, succinic acid, phosphoric acid, sodium hydroxide, and sodium carbonate). Other hair care additives are described in the "COSMETIC INGREDIENT DICTIONARY" published by "THE COSMETIC TOILETRY AND FRAGRANCE ASSOCIATION INC.", U.S. Patent No. 5,009,880, and Kirk-Othmer, Encyclopedia of Chemical Technology, 4th Ed., Vol. 12, John Wiley & Sons, pages 881-917.

The pH of the hair-treating composition is typically 3 to 9, preferably from 6 to 7. For application to human hair on a person, Tₘ is preferably at least 40°C so that the hair does not become unset at high ambient temperatures, but generally is no more than 60°C, so the hair can be reset with a hair dryer or curling iron. A particular advantage of the present invention is that the coatings can be applied to hair, and the hair can be repeatedly reset. Thus, the hair can be set in a desired configuration, and then by heating the crystalline polymer to a temperature greater than Tₘ, the hair can be reset into a second desired configuration. However, when the hair is on a wig or a doll, it can be desirable to set the hair more permanently. In such a case, Tₘ is sufficiently high, preferably at least 60°C. Alternatively, or in addition, the composition can be externally crosslinked, as described above.

When a coating is formed on human hair, it is generally desirable that the coating can be easily removed by washing with water. This can be achieved by using a monomer component which contains (a) an XLC as described above, and/or (b) at least 0.5% of at least one amphoteric monomer, and/or (c) a carboxyl-containing monomer component as described above.

The invention is illustrated in the accompanying drawings, in which Figures 1-6 are DSC curves for polymers prepared in the Examples, as further described below, and Figures 7 and 8 are schematic illustrations of a particulate coating 10, made up of coalesced but still identifiable particles 14, on a substrate 12, which is a fiber or a seed, respectively.

### EXAMPLES

The invention is illustrated by the following examples, some of which are comparative examples.

### Examples 1-16

Examples 1-16 illustrate the preparation of aqueous polymer dispersions. Examples 1, 5, 6, 9 and 10 are less preferred, in that the crystalline polymer does not have a single, sharply defined melting temperature.

The following abbreviations are used in Examples 1-16 (and in the later Examples): C6A, hexyl acrylate (amorphous hydrophobic monomer); C12A, dodecyl acrylate, C14A, tetradecyl acrylate, C16A, hexadecyl acrylate, and C18A, octadecyl acrylate (all crystalline hydrophobic monomers); C6DA, 1,6-hexanediol diacrylate, and CD 14A, 1,14-tetradecanediol diacrylate (both crosslinking monomers); AA, acrylic acid, and MA, methacrylic acid (both hydrophilic monomers); C12SH, dodecyl mercaptan (chain transfer agent); SP, sodium persulfate (initiator); PP, potassium persulfate (initiator); SBC, sodium bicarbonate (buffer); EAC, ethyl acetate (cosolvent); IPA, isopropanol (cosolvent).

The following are surfactants: SDS, sodium dodecyl sulfate; 15-S-7, 15-S-9 and 15-S-40 are C₁₁-C₁₅ secondary alcohol ethoxylates which are available from Union Carbide under the trade names Tergitol 15-S-7, 15-S-9 and 15-S-40, respectively; P1059, an amine salt of dodecyl benzene sulfonic acid which is available from Witco under the trade name Witconate P1059; EMC, sodium dioctyl sulfosuccinate which is available from Witco under the trade name EMCOL DOSS; SLS-R which is a 30% by weight lauryl sulfate solution in water available from Lonza, Inc under the trade name Carsonol SLS-R; and A-102 which is a disodium ethoxylated alcohol half ester of sulfosuccinic acid available from Cytec under the trade name AEROSOL A-102.

The amounts of each ingredient used in the Examples are shown in Tables 1-3.

Examples 1-8 illustrate how addition of cosolvent and high surfactant concentrations reduce the heterogeneity of emulsion polymerization reaction products prepared by using at least two different monomer components. Examples 1-4 show the effect of the addition of cosolvent in the presence of high surfactant concentrations on the heterogeneity of the polymerization reaction products. Examples 5-8 show the effect of the addition of cosolvent in the presence of low surfactant concentrations on the heterogeneity.

### Examples 1-4

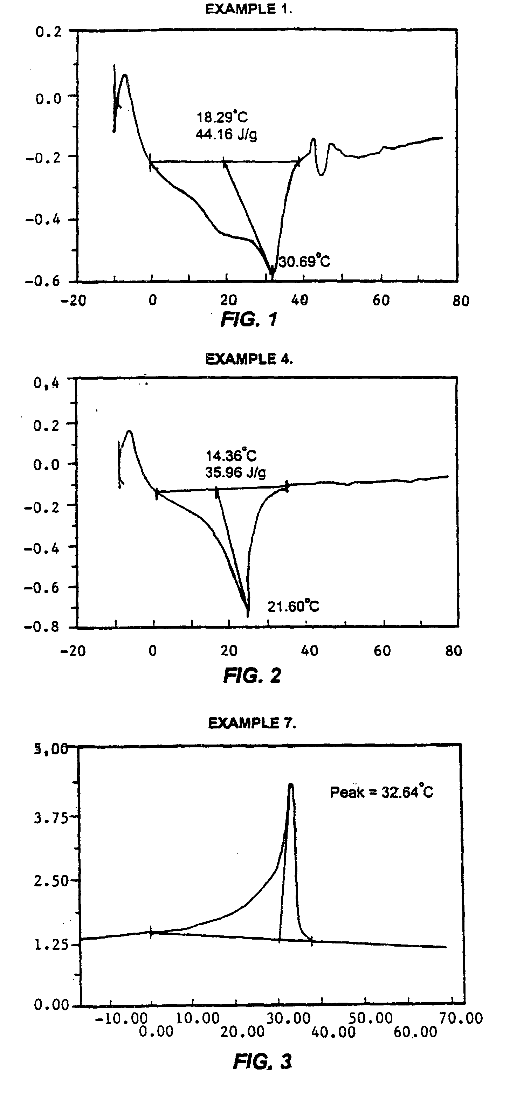

Polymers consisting of four monomer components (C16A, C6A, CD14A and MA) were prepared in the presence of a constant amount of surfactant (3.2%) and variable amounts of cosolvent as shown in Table 1. The emulsion polymerization components were mixed and degassed for 30 minutes. The polymerization reaction proceeded for 4 hours at 70°C under nitrogen. The polymer prepared in comparative Example 1 in the absence of cosolvent exhibited a broad profile with two peaks on a DSC thermograph as shown in Figure 1. The polymer prepared in Example 2 in the presence of 5% by weight ethanol shows a much sharper DSC peak and a lower Tₘ. The polymer prepared in Example 3 in the presence of 5% by weight ethyl acetate similarly exhibits a sharp peak. Addition of higher concentrations of ethyl acetate above 5% only has a slight effect on the sharpness of the DSC peak and the Tₘ of the polymer product as shown in Figure 2.

### Examples 5-8

Polymers consisting of three monomer components (C16A, C6A, and MA) were prepared in the presence of a constant, but lower, amount of surfactant (1.6%) and variable amounts of cosolvent as shown in Table 1. The emulsion polymerization components were mixed and degassed for 30 minutes. The polymerization reaction proceeded for 4 hours at 70°C under nitrogen. The polymer prepared in comparative Example 5 in the absence of cosolvent exhibits multiple DSC peaks. The polymer prepared in comparative Example 6 in the presence of 5% by weight ethyl acetate shows a single DSC peak and a lower Tₘ. The polymer prepared in Example 7 in the presence of 10% by weight ethyl acetate exhibits a sharp peak (Figure 3). Addition of higher concentrations of ethyl acetate above 5% only has a slight effect on the sharpness of the DSC peak and the Tₘ of the polymer product.

**TABLE 1**

| | Ex. 1 | Ex. 2 | Ex. 3 | Ex. 4 | Ex. 5 | Ex. 6 | Ex. 7 | Ex. 8 |
|---|---|---|---|---|---|---|---|---|
| H₂O | 200 g | 190 g | 190 g | 180 g | 200 g | 190 g | 180 g | 160 g |
| C16A | 70 g | 70 g | 70 g | 70 g | 70 g | 70 g | 70 g | 70 g |
| C6A | 25 g | 25 g | 25 g | 25 g | 25 g | 25 g | 25 g | 25 g |
| MA | 5 g | 5 g | 5 g | 5 g | 5 g | 5 g | 5 g | 5 g |
| C14DA | 2 g | 1.5 g | 1.5 g | 1.5 g | - | - | - | - |
| 15-S-9 | 5 g | 5 g | 5 g | 5 g | 2.5 g | 2.5 g | 2.5 g | 2.5 g |
| P1059 | 5 g | 5 g | 5 g | 5 g | 2.5 g | 2.5 g | 2.5 g | 2.5 g |
| PP | 0.4 g | 0.4 | 0.4 g | 0.4 g | 0.4 g | 0.4 g | 0.4 g | 0.4 g |
| EAc | - | - | 10 g | 20 g | - | 10 g | 20 g | 40 g |
| Ethanol | - | 10 g | - | - | - | - | - | - |
| DSC | broad peak | sharp peak | sharp peak | sharp peak | multiple peaks | broad peak | sharp peak | sharp peak |
| Tₘ (°C) | 30.7 | 22.5 | 21.9 | 21.6 | 34.8 | 33.0 | 32.6 | 22.4 |

### Examples 9-12

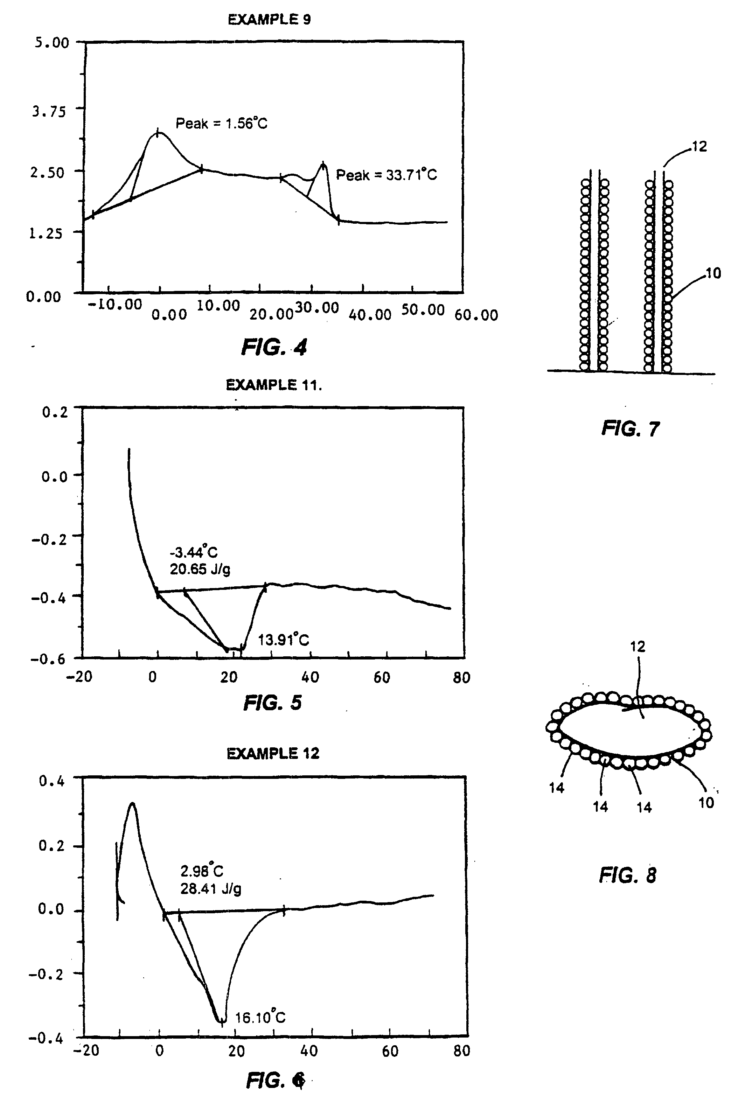

Polymers consisting of two SCC monomer components (C16A and C12A) and MA were prepared. Both C16A and C12A -based polymers are side chain crystalline polymers and can be detected by DSC whereas C6A polymers are amorphous and not detectable. The polymers were prepared in the presence of varying concentrations of surfactants and cosolvents as shown in Table 2. The emulsion polymerization components were mixed and degassed for 30 minutes. The polymerization reaction proceeded for 4 hours at 70°C under nitrogen. The polymer prepared in comparative Example 9 in the presence of a low surfactant concentration (1.2%) in the absence of cosolvent exhibited two distinct peaks with Tₘ's at 1.6°C and 33.7°C as shown in Figure 4. The polymer prepared in comparative Example 10 in the presence of the same amount of surfactant and 5% by weight ethyl acetate shows a broad peak with three peaks with the predominant polymer species having a Tₘ of 2.7°C. The polymer of Example 11 was prepared in the presence of higher concentrations of surfactant (2.4%). Increasing the surfactant concentration produces a polymer product with a sharp Tₘ at 13.9°C as shown in Figure 5. Example 12 shows the use of different monomer components (C14A and C12A) in a reaction mixture similar to that of Example 11. Once again only one peak is observed as shown in Figure 6.

**TABLE 2**

| | Ex. 9 | Ex. 10 | Ex. 11 | Ex. 12 |
|---|---|---|---|---|
| H₂O | 200 g | 190 g | 400 g | 400 g |
| C16A | 35 g | 35 g | 70 g | - |
| C14A | - | - | - | 95 g |
| C12A | 60 g | 60 g | 120 g | 95 g |
| MA | 5 g | 5 g | 10 g | 10 g |
| 15-S-9 | 2 g | 2 g | 8 g | 8 g |
| DOSS | 2 g | 2 g | 8 g | 8 g |
| PP | 2 g | 0.4 g | 1.6 g | 1.5 g |
| EAc | - | 10 g | - | - |
| C12SH | - | - | - | 0.1 g |
| DSC | two peaks | broad peak | sharp peak | sharp peak |
| Tₘ (°C) | 2, 33.7 | 2.7 | 13.9 | 16.1 |

### Examples 13-15

These examples show the large scale preparation of copolymers using the method described in this invention. Reaction components are shown in Table 3.

In Example 13 the emulsion polymerization components were mixed and degassed for 30 minutes. The polymerization reaction proceeded for 4 hours at 70°C under nitrogen. The polymer was polymerized in the presence of a total surfactant concentration of 2.9% in the presence of 20 % cosolvent. The polymer product exhibited a peak with a Tₘ of 11.3°C.

In Example 14 the emulsion polymerization components were mixed and degassed for 30 minutes. Part of the polymerization reaction mixture (25%) was metered into a 1 liter resin kettle for 10 minutes at a rate of 12 ml/minute. The mixture was polymerized for 15 minutes at 80°C. The rest of the monomer solution was metered over 75 minutes and the polymerization reaction proceeded for 2 more hours under nitrogen. The polymer product exhibited a peak with a Tₘ of 9°C.

In Example 15 all the polymerization components, except for the initiator, were mixed and degassed. Once the polymerization components were heated to 60°C, degassed initiator was added to the reaction mixture. The reaction proceeded for two hours while maintaining the internal temperature of the mixture at 70°C. After two hours more initiator was added to the reaction mixture to drive polymerization to completion. The reaction proceeded for a total of 5 hours. The reaction was cooled overnight. After cooling the product was neutralized to pH 7 with ammonium hydroxide and filtered with a 63 micron sieve. The polymer exhibited a single sharp peak at 9.2°C.

**TABLE 3**

| | Ex. 13 | Ex. 14 | Ex. 15 |
|---|---|---|---|
| H₂O | 800 g | 320 g | 4308 g |
| C14A | 405 g | 160 g | 2080 g |
| C6A | 70 g | 30 g | 390 g |
| C6DA | - | 1.4 g | 18.2 g |
| MA | 25 g | 5 g | 130 g |
| AA | - | 10 g | - |
| A-102 | 20 g | 8 g | 104 g |
| SLS | 10 g | 4 g | 52 g |
| 15-S-7 | 10 g | 4 g | 52 g |
| 15-S-40 | 2.5 g | 4 g | 13 g |
| PP | 2 g | 0.8 g | 15.6 g |
| SBC | 2 g | 0.8 g | 10.4 |
| IPA | 200 g | 80 g | 1040 g |
| DSC | sharp peak | sharp peak | sharp peak |
| Tₘ (°C) | 11.2 | 9.1 | 9.2 |

### Example 16

A polymer was prepared with 2-acrylamido-2-methylpropane sulfonic acid (AMPS, available from Lubrizol) as follows. C16A (31.5 g), AMPS (6 g of a 50% aq. solution), water (80 g), isopropanol (20 g), Aerosol A-102 (2 g), Carsonol SLS-R (1 g) were mixed together and purged with N₂ for 20 min. The bottle was then capped and heated to 80°C, and 0.4 g of potassium persulfate was added. The reaction was carried out at 80°C for 3 h. A bluish tan emulsion was formed, cooled overnight and neutralized with ammonium hydroxide.

### Examples 17-20

Examples 17-20 illustrate the preparation and use of aqueous polymer dispersions for coating hair. The molecular weights (which are given as Mₙ values, i.e. number average molecular weights, or M_{w} values, i.e. weight average molecular weights) were measured by the standard GPC technique in tetrahydrofuran, against polystyrene standards. The following additional abbreviations are used in these and later Examples. C4A, butyl acrylate (amorphous monomer); ESH, mercaptoethanol (chain transfer agent); AIBN, azobisisobutyronitrile (initiator); ESP, t-amylperoxy 2-ethyl hexanoate which is available from WITCO under the trade name Esperox 570 (initiator); TERG, a C₁₁-C₁₅ secondary alcohol ethoxylate which is available from Union Carbide under the trade name Tergitol 15-S-9 (surfactant).

### Example 17

A mixture of C18A (1865 g), MA (150 g)) and C12SH (1 g) was added over a period of 20 minutes to a reaction vessel which was purged with nitrogen, stirred, and maintained at 100°C. Over the same period, ESP (10 g) was added separately. After 5 hours, the temperature was raised to 120°C, and heating continued for a total reaction time of 23 hours. The resulting white polymer had an M_{w} of 198,000, Mₙ of 26,000, Tₒ of 43°C and Tₚ of 45°C.

### Example 18

A sample of the polymer produced in Example 17 was finely ground and was added to a mixture of water (90 ml), TERG (1.25 g) and P-1059 (1.25 g) which was maintained at 80°C in a stirred reaction vessel. After 2 hours of mixing, a stable emulsion had been produced.

### Example 19

A mixture of C18A (95 g), MA (5 g) and C12SH (0.05 g) was added to a mixture of water (200 ml), TERG (5 g) and EMC (5 g) which was maintained at 80°C in a stirred reaction vessel. After degassing with nitrogen for 30 minutes, PP (0.4 g) was added. Heating was maintained for 4 hours, and after the reaction mixture had cooled slowly to room temperature, it was neutralized with 10% aqueous ammonium hydroxide. The resulting milky white polymer had a Tₒ of 43°C, Tₚ of 47°C, and a M_{w} above 1,500,000.

### Example 20

The procedure of Examples 18 and 19 were followed to generate a water based emulsion of SCC polymer. The milky white formulation was applied to the hair of a doll through a pump spray bottle. The hair was then heated with a hair dryer, evaporating the water and melting the SCC polymer. While the polymer was in the molten state, the doll's hair was shaped in a desired configuration.

When the doll's hair needed to be set in another configuration, the hair was first reheated to melt the composition retained in the hair. The hair was then reshaped in the new configuration. This process was repeated several times, and each time the doll's hair retained the new desired configuration.

## Claims

1. A method of treating human hair, the method comprising
(A) applying to the human hair a coating composition which has a pH of 3 to 9, and which comprises water, a surfactant, and, dispersed in the water, solid particles having an average particle size of less than 0.5 micron, and comprising a crystalline polymer which
(1) has a melting point Tₘ and an onset-of-melting temperature, Tₒ , such that Tₘ -Tₒ is less than 20 °C, and
(2) is derived from a monomer component comprising
(a) a hydrophobic monomer component comprising
(i) a first hydrophobic monomer which is such that a homopolymer thereof is a side chain crystalline (SCC) polymer having a melting point T_{c}, and (ii) a second hydrophobic monomer which is such that a homopolymer thereof is amorphous and has a glass transition point T_{g} which is less than T_{c}; and is from -50 to 120 °C, and
(b) a hydrophilic monomer component comprising a hydrophilic monomer,
one or both of the hydrophobic monomer component and the hydrophilic monomer component containing at least one monomer selected from one or more of
(i) crosslinking monomers having at least two polymerizable groups,
(ii) amphoteric monomers in amount at least 0.5% by weight, based on weight of the monomer component, and
(iii) monomers containing carboxyl groups, the carboxyl groups being at least 75% neutralized;
and
(B) evaporating water from the composition to form a solid polymeric coating on the human hair.

2. A method according to claim 1 wherein the coating composition contains 2 to 50% by weight, based on the weight of the composition, of the solid particles.

3. A method according to claim 1 or 2 wherein Tₘ is 40 to 60 °C, and Tₘ - Tₒ is less than 15 °C.

4. A method according to any one of the preceding claims wherein the hydrophobic monomer component contains 5 to 40% by weight, based on the weight of the hydrophobic monomer component, of the second hydrophobic monomer.

5. A method according to any one of the preceding claims wherein the monomer component contains 2 to 10% by weight, based on the weight of the monomer component, of the hydrophilic monomer components, and the ratio by weight of the hydrophobic monomer component to the hydrophilic monomer component is 10:1 to 50:1.

6. A method according to any one of the preceding claims wherein the composition contains less than 1% of unreacted monomer, based on the weight of the polymer.

7. _{A} method according to any one of the preceding claims wherein the the first hydrophobic monomer comprises a linear polymethylene group containing 12 to 50 carbon atoms or a linear perfluorinated or substantially perfluorinated polymethylene group containing 6 to 50 carbon atoms, and the second hydrophobic monomer comprises a linear or branched chain alkyl, hydroxyalkyl or alkoxyalkyl acrylate, methacrylate, acrylamide, or methacrylamide or a vinyl ester.

8. A method according to any one of the preceding claims wherein the surfactant comprises an anionic surfactant having an HLB number of 20 to 40 and a nori-ionic surfactant having an HLB number of 5 to 35.

9. A coating composition which is suitable for use in step (A) of any one of the preceding claims, which has a pH of 3 to 9, and which comprise water, a surfactant, and, dispersed in the water, solid particles having an average particle size of less than 0.5 micron and comprising a crystalline polymer which
(1) has a melting point Tₘ and an onset-of-melting temperature, Tₒ , such that Tₘ -Tₒ is less than 20 °C,
(2) is derived from a monomer component comprising
(a) a hydrophobic monomer component comprising
(i) a first hydrophobic monomer which is such that a homopolymer thereof is a side chain crystalline (SCC) polymer having a melting point T_{c}, and
(ii) a second hydrophobic monomer which is such that a homopolymer thereof is amorphous and has a glass transition point T_{g} which is less than T_{c}, and is from -50 to 120 °C, and
(b) a hydrophilic monomer component comprising a hydrophilic monomer,
one or both of the hydrophobic monomer component and the hydrophilic monomer component containing at least one monomer selected from one or more of
(i) crosslinking monomers having at least two polymerizable groups,
(ii) amphoteric monomers in amount at least 0.5% by weight, based on weight of the monomer component, and
(iii)monomers containing carboxyl groups, the carboxyl groups being at least 75% neutralized.

10. A composition according to claim 9 which contains an additive selected from the group consisting of sequestering agents, hair-conditioning agents, anti-dandruff agents, reducing agents, bleaching agents, perfumes, pearlescent aids, preservatives, thickeners, coloring agents, and pH adjusting agents.

## Patentansprüche

1. Verfahren zur Behandlung von menschlichem Haar, bei dem
(A) eine Beschichtungszusammensetzung mit einem pH von 3 bis 9 auf das menschliche Haar aufgebracht wird, die Wasser, ein Tensid und in dem Wasser dispergierte, feste Teilchen umfaßt, die eine durchschnittliche Teilchengröße von weniger als 0,5 µm haben und ein kristallines Polymer umfassen, das
(1) einen Schmelzpunkt Tₘ und eine Schmelzbeginntemperatur Tₒ hat, so daß Tₘ-Tₒ weniger als 20°C ist, und
(2) abgeleitet ist von einer Monomerkomponente, die
(a) eine hydrophobe Monomerkomponente, die
(i) ein erstes hydrophobes Monomer, das so ist, daß ein Homopolymer daraus ein kristallines Seitenkettenpolymer (SCC) mit einem Schmelzpunkt T_{c} ist, und
(ii) ein zweites hydrophobes Monomer umfaßt, das so ist, daß ein Homopolymer daraus amorph ist und eine Glasübergangstemperatur T_{g} aufweist, die geringer als T_{c} ist und von -50 bis 120°C beträgt, und
(b) eine hydrophile Monomerkomponente umfaßt, die ein hydrophiles Monomer umfaßt,
wobei die hydrophobe Monomerkomponente oder die hydrophile Monomerkomponente oder beide mindestens ein Monomer ausgewählt aus einem oder mehreren von
(i) Vernetzungsmonomeren mit mindestens zwei polymerisierbaren Gruppen,
(ii) amphoteren Monomeren in einer Menge von mindestens 0,5 Gew.-%, bezogen auf das Gewicht der Monomerkomponente, und
(iii) Monomeren, die Carboxylgruppen enthalten, wobei die Carboxylgruppen zu mindestens 75 % neutralisiert sind, enthalten und
(B) das Wasser aus der Zusammensetzung unter Bildung einer festen polymeren Beschichtung auf dem menschlichen Haar verdunstet wird.

2. Verfahren nach Anspruch 1, bei dem die Beschichtungszusammensetzung, bezogen auf das Gewicht der Zusammensetzung, 2 bis 50 Gew.-% der festen Teilchen enthält.

3. Verfahren nach Anspruch 1 oder 2, bei dem Tₘ 40 bis 60°C ist und Tₘ-Tₒ weniger als 15°C ist.

4. Verfahren nach einem der vorangehenden Ansprüche, bei dem die hydrophobe Monomerkomponente, bezogen auf das Gewicht der hydrophoben Monomerkomponente, 5 bis 40 Gew.-% des zweiten hydrophoben Monomers enthält.

5. Verfahren nach einem der vorangehenden Ansprüche, bei dem die Monomerkomponente, bezogen auf das Gewicht der Monomerkomponente, 2 bis 10 Gew.-% der hydrophilen Monomerkomponente enthält und das Gewichtsverhältnis der hydrophoben Monomerkomponente zu der hydrophilen Monomerkomponente 10:1 bis 50:1 beträgt.

6. Verfahren nach einem der vorangehenden Ansprüche, bei dem die Zusammensetzung, bezogen auf das Gewicht des Polymers, weniger als 1% nicht umgesetztes Monomer enthält.

7. Verfahren nach einem der vorangehenden Ansprüche, bei dem das erste hydrophobe Monomer eine lineare Polymethylengruppe mit 12 bis 50 Kohlenstoffatomen oder eine lineare perfluorierte oder im wesentlichen perfluorierte Polymehtylengruppe mit 6 bis 50 Kohlenstoffatomen und das zweite hydrophobe Monomer ein lineares oder verzweigtkettiges Alkyl-, Hydroxyalkyl- oder Alkoxyalkylacrylat, -methacrylat, -acrylamid oder -methacrylamid oder einen Vinylester umfaßt.

8. Verfahren nach einem der vorangehenden Ansprüche, bei dem das Tensid ein anionisches Tensid mit einer HLB-Zahl von 20 bis 40 und ein nicht-ionisches Tensid mit einer HLB-Zahl von 5 bis 35 umfaßt.

9. Beschichtungszusammensetzung, die zur Verwendung in Stufe
(A) von jedem der vorangehenden Ansprüche geeignet ist, die einen pH von 3 bis 9 hat und Wasser, ein Tensid und in dem Wasser dispergierte, feste Teilchen umfaßt, die eine durchschnittliche Größe von weniger als 0,5 µm haben und ein kristallines Polymer umfassen, das
(1) einen Schmelzpunkt Tₘ und eine Schmelzbeginntemperatur Tₒ hat, so daß Tₘ-Tₒ weniger als 20°C ist, und
(2) abgeleitet ist von einer Monomerkomponente, die
(a) eine hydrophobe Monomerkomponente, die
(i) ein erstes hydrophobes Monomer, das so ist, daß ein Homopolymer daraus ein kristallines Seitenkettenpolymer (SCC) mit einem Schmelzpunkt T_{c} ist, und
(ii) ein zweites hydrophobes Monomer umfaßt, das so ist, daß ein Homopolymer daraus amorph ist und eine Glasübergangstemperatur T_{g} aufweist, die geringer als T_{c} ist und von -50 bis 120°C beträgt, und
(b) eine hydrophile Monomerkomponente umfaßt, die ein hydrophiles Monomer umfaßt,
wobei die hydrophobe Monomerkomponente oder die hydrophile Monomerkomponente oder beide mindestens ein Monomer ausgewählt aus einem oder mehreren von
(i) Vernetzungsmonomeren mit mindestens zwei polymerisierbaren Gruppen,
(ii) amphoteren Monomeren in einer Menge von mindestens 0,5 Gew.-%, bezogen auf das Gewicht der Monomerkomponente, und
(iii) Monomeren, die Carboxylgruppen enthalten,
wobei die Carboxylgruppen zu mindestens 75 % neutralisiert sind, enthalten.

10. Zusammensetzung nach Anspruch 9, die ein Additiv ausgewählt aus der Gruppe bestehend aus Sequestriermitteln, Haarkonditioniermitteln, Antischuppenmitteln, Reduktionsmitteln, Bleichmitteln, Parfüms, Perlmutterglanzhilfsmitteln, Konservierungsmitteln, Verdickungsmitteln, Färbemitteln und pH-Regulierungsmitteln enthält.

## Revendications

1. Procédé de traitement de cheveux humains, le procédé comprenant:
(A) l'application aux cheveux humains d'une composition d'enrobage qui a un pH de 3 à 9, et qui comprend de l'eau, un agent tensio-actif, et, dispersées dans l'eau, des particules solides ayant une dimension moyenne de particule de moins de 0,5 micron, et comprenant un polymère cristallin, lequel :
(1) a un point de fusion Tₘ et une température de début de fusion, Tₒ, telle que Tₘ - Tₒ est moins de 20°C ; et
(2) est issu d'un composant monomère comprenant :
(a) un composant monomère hydrophobe comprenant :
(i) un premier monomère hydrophobe qui est tel qu'un homopolymère de celui-ci est un polymère cristallin à chaînes latérales (SCC) ayant un point de fusion T_{c} ; et
(ii) un second monomère hydrophobe qui est tel qu'un homopolymère de celui-ci est amorphe et a un point de transition vitreuse T_{g} qui est inférieur à T_{c}, et est de -50 à 120°C ; et
(b) un composant monomère hydrophile comprenant un monomère hydrophile,
l'un parmi le composant monomère hydrophobe et le composant monomère hydrophile ou les deux contenant au moins un monomère choisi parmi un ou plusieurs de :
(i) monomères réticulants ayant au moins deux groupes polymérisables ;
(ii) monomères amphotères en quantité d'au moins 0,5% en poids, sur la base du poids du composant monomère ; et
(iii)monomères contenant des groupes carboxyle, les groupes carboxyle étant neutralisés à au moins 75% ; et
(B) l'évaporation de l'eau à partir de la composition pour former un enrobage polymère solide sur les cheveux humains.

2. Procédé selon la revendication 1, dans lequel la composition d'enrobage contient 2 à 50% en poids, sur la base du poids de la composition, des particules solides.

3. Procédé selon l'une des revendications 1 et 2, dans lequel Tₘ est de 40 à 60°C, et Tₘ - Tₒ est moins de 15°C.

4. Procédé selon l'une quelconque des revendications précédentes, dans lequel le composant monomère hydrophobe contient 5 à 40% en poids, sur la base du poids du composant monomère hydrophobe, du second monomère hydrophobe.

5. Procédé selon l'une quelconque des revendications précédentes, dans lequel le composant monomère contient 2 à 10% en poids, sur la base du poids du composant monomère, du composant monomère hydrophile, et le rapport en poids du composant monomère hydrophobe au composant monomère hydrophile est de 10:1 à 50:1.

6. Procédé selon l'une quelconque des revendications précédentes, dans lequel la composition contient moins de 1% de monomère n'ayant pas réagi, sur la base du poids du polymère.

7. Procédé selon l'une quelconque des revendications précédentes, dans lequel le premier monomère hydrophobe comprend un groupe polyméthylène linéaire contenant 12 à 50 atomes de carbone ou un groupe polyméthylène perfluoré ou sensiblement perfluoré linéaire, contenant 6 à 50 atomes de carbone, et le second monomère hydrophobe comprend un acrylate, méthacrylate, acrylamide ou méthacrylamide d'alkyle, d'hydroxyalkyle ou d'alcoxyalkyle à chaîne droite ou ramifiée, ou un ester vinylique.

8. Procédé selon l'une quelconque des revendications précédentes, dans lequel l'agent tensio-actif comprend un agent tensio-actif anionique ayant un indice HLB de 20 à 40 et un agent tensio-actif non-ionique ayant un indice HLB de 5 à 35.

9. Composition d'enrobage qui est appropriée pour être utilisée à l'étape (A) de l'une quelconque des revendications précédentes, qui a un pH de 3 à 9, et qui comprend de l'eau, un agent tensio-actif, et, dispersées dans l'eau, des particules solides ayant une dimension moyenne de particule de moins de 0,5 micron et comprenant un polymère cristallin, lequel :
(1) a un point de fusion Tₘ et une température de début de fusion, Tₒ, telle que Tₘ - Tₒ est moins de 20°C ;
(2) est issu d'un composant monomère comprenant :
(a) un composant monomère hydrophobe comprenant :
(i) un premier monomère hydrophobe qui est tel qu'un homopolymère de celui-ci est un polymère cristallin à chaînes latérales (SCC) ayant une point de fusion T_{c} ; et
(ii) un second monomère hydrophobe qui est tel qu'un homopolymère de celui-ci est amorphe et a un point de transition vitreuse T_{g} qui est inférieur à T_{c}, et est de -50 à 120°C ; et
(b) un composant monomère hydrophile comprenant un monomère hydrophile,
l'un parmi le composant monomère hydrophobe et le composant monomère hydrophile ou les deux contenant au moins un monomère choisi parmi un ou plusieurs de :
(i) monomères réticulants ayant au moins deux groupes polymérisables ;
(ii) monomères amphotères en quantité d'au moins 0,5% en poids, sur la base du poids du composant monomère ; et
(iii)monomères contenant des groupes carboxyle, les groupes carboxyle étant neutralisés à au moins 75%.

10. Composition selon la revendication 9, qui contient un additif choisi dans le groupe constitué par les agents séquestrants, les agents de conditionnement capillaire, les agents anti-pelliculaires, les agents réducteurs, les agents décolorants, les parfums, les auxiliaires nacrants, les conservateurs, les épaississants, les agents colorants et les agents d'ajustement de pH.
